Europäisches Patentamt

(19) European Patent Office

Office européen des brevets

(11) Numéro de publication: **0 057 624**
**B1**

(12) FASCICULE DE BREVET EUROPEEN

(45) Date de publication du fascicule du brevet:
01.08.84

(51) Int. Cl.³: **B 01 D 53/34**, A 61 L 9/015

(21) Numéro de dépôt: **82400071.5**

(22) Date de dépôt: **15.01.82**

(54) **Procédé de désodorisation d'air pollué.**

(30) Priorité: **19.01.81 FR 8100906**

(43) Date de publication de la demande:
**11.08.82 Bulletin 82/32**

(45) Mention de la délivrance du brevet:
**01.08.84 Bulletin 84/31**

(84) Etats contractants désignés:
**BE CH DE GB LI NL SE**

(56) Documents cités:
**FR - A - 706 750**
**FR - A - 2 150 966**
**FR - A - 2 229 447**
**FR - A - 2 271 862**
**US - A - 3 969 479**

**CHEMICAL ABSTRACTS, vol. 92, no. 6, 11 février 1980,**
**page 302, ref. 46756d Columbus Ohio (US)**
**JAPANESE PATENTS GAZETTE, partie I, Chemical**
**Week X24-Unexamined, 21 Juillet 1976, Derwent**
**publications Londres (GB), ref. 44446x/24**

(73) Titulaire: **Etablissement public dit: CHARBONNAGES**
**DE FRANCE, 9, Avenue Percier, F-75008 Paris (FR)**

(72) Inventeur: **Chelu, Gérard, 23, rue Le Chatelier,**
**F-60550 Verneuil en Halatte (FR)**

(74) Mandataire: **Chevallier, Robert Marie Georges, Cabinet**
**BOETTCHER 23, rue La Boétie, F-75008 Paris (FR)**

ACTORUM AG

## Description

L'invention concerne un procédé de désodorisation d'air pollué contenant des résidus gazeux malodorants d'origine organique, dans lequel l'air à désodoriser est traité par l'ozone et lavé dans un laveur au moyen d'une solution aqueuse.

Dans de nombreuses usines, par exemple les usines d'équarrissage, les fondoirs de suif, les usines de traitement d'eaux, la présence de composés malodorants dans les effluents gazeux est une cause importante de problèmes de voisinage. Pour faire disparaître ces odeurs, on a déjà proposé de nombreux procédés tels que le lavage oxydant avec de multiples variantes, l'adsorption sur charbon actif, l'oxydation thermique à haute température ou catalytique à température plus modérée, l'oxydation par l'ozone à température ambiante éventuellement complétée par un lavage comme dans le procédé rappelé ci-dessus. La plupart de ces procédés sont insuffisamment efficaces ou d'un coût prohibitif.

Les principaux composés responsables des odeurs nauséabondes sont des composés soufrés, notamment l'hydrogène sulfuré et des mercaptans tels le méthylmercaptan, l'ammoniac et des amines, telle la triméthylamine, des aldéhydes tels que l'acroléine, le valéraldéhyde, le butyraldéhyde, des cétones, telles que la méthyléthylcétone. Si la plupart des composés soufrés et ammoniaqués réagissent avec l'ozone, il n'en est pas de même des aldéhydes.

Un but de l'invention est de proposer un procédé permettant de neutraliser en un seul traitement et à température ambiante les composés malodorants, et plus particulièrement ceux des familles rappelées ci-dessus, que l'analyse permet ou non d'identifier. Un autre but de l'invention est de proposer un procédé d'une grande efficacité, facile à mettre en œuvre, peu consommateur en agent oxydant, grâce à un réglage de celui-ci en fonction des besoins. Un procédé connu de désodorisation d'air pollué par des gaz malodorants d'origine organique comprenant l'utilisation d'ozone et le lavage de cet air à l'aide d'une solution aqueuse de peroxyde d'hydrogène est décrit par JP-A N° 79.101764.

En mettant en œuvre un procédé de ce type et en utilisant une solution aqueuse de peroxyde d'hydrogène dans le rapport initial de 0,5/100 à 10/100 en masse de la solution, de préférence de l'ordre de 5/100, le titulaire a constaté que la désodorisation de l'air pollué est complète avec neutralisation, même à température ambiante, de tous les composés précités. Mais le titulaire a fait, en outre, une constatation surprenante, à savoir que, si on recycle la solution aqueuse de peroxyde d'hydrogène, c'est-à-dire d'eau oxygénée, l'injection d'ozone suffit à maintenir le titre de cette eau oxygénée à sa valeur initiale; il n'est alors plus besoin de prévoir d'appoint de peroxyde d'hydrogène, le simple appoint d'ozone étant suffisant. Il est possible, sans que ce soit une explication scientifiquement établie, que le procédé de l'invention favorise la formation radicalaire de peroxydes dans la solution aqueuse. Quoi qu'il en soit, moyennant l'introduction de peroxyde d'hydrogène dans la seule phase liquide initiale, il n'est plus besoin d'en introduire par la suite, ce qui rend le procédé très économique, tout en permettant cependant de neutraliser les composés, tels que les aldéhydes, que le traitement à l'ozone + eau de lavage ne permet pas de neutraliser.

L'addition de peroxyde d'hydrogène, selon l'invention, se distingue des usages précédents de ce composé qu'il est d'usage d'introduire dans la phase liquide ou solide des déchets, produits ou autres immondices comme dans les brevets français Nos 2187729, 2281908 ou 2416016 où on utilise et/ou consomme de l'eau oxygénée jusqu'à environ 500 ppm. Dans l'invention, à part l'apport initial, on ne consomme pas de peroxyde d'hydrogène apporté.

Il est donc important, selon l'invention, que la solution aqueuse de lavage soit mise en circulation en circuit fermé.

Selon un mode de réalisation, on introduit l'ozone dans l'air pollué avant son lavage par la solution aqueuse, comme il est connu en soi.

Mais, selon un mode de réalisation préféré de l'invention, on introduit l'ozone dans le circuit de lavage, par exemple par barbotage dans la solution aqueuse de lavage séjournant dans un réservoir-tampon.

Selon un mode de réalisation économique de l'invention, on récupère la phase gazeuse non absorbée à la suite de l'introduction de l'ozone dans la solution aqueuse de lavage du réservoir-tampon, et on introduit ladite phase gazeuse récupérée dans l'air pollué avant son lavage par la solution aqueuse.

Selon une caractéristique intéressante de l'invention, on règle le débit d'ozone introduit par asservissement à une valeur de consigne de la quantité d'ozone dosée dans l'air effluent sortant du laveur.

De cette façon, on obtient un réglage quasi stœchiométrique de la quantité d'ozone introduit, et donc consommé, ce qui permet une conduite particulièrement économique du procédé.

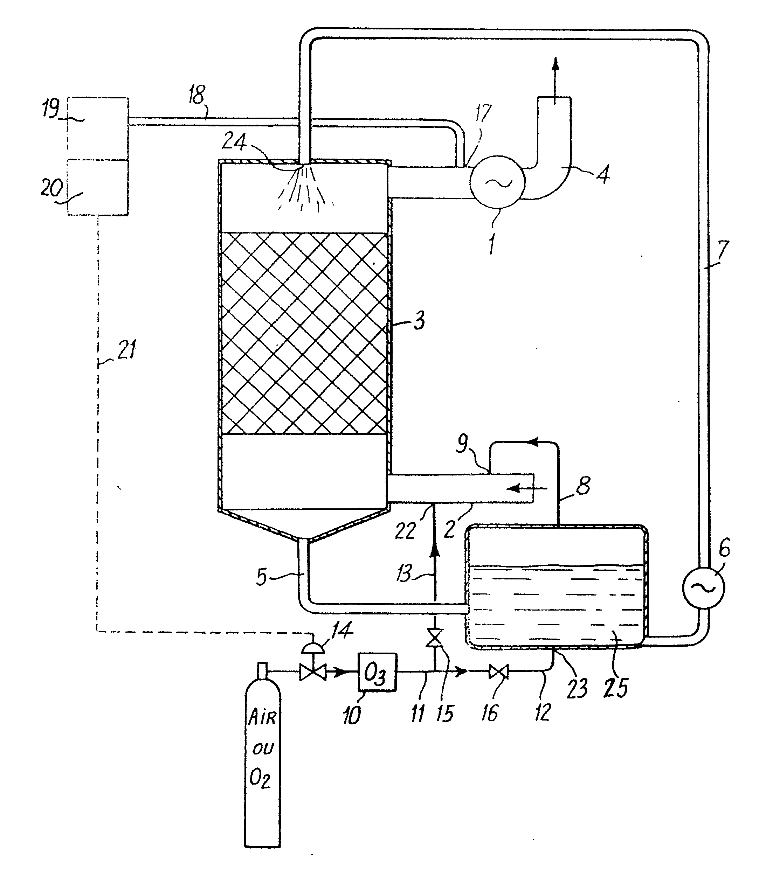

D'autres caractéristiques et avantages ressortiront de la descritpion, qui sera donnée ci-après uniquement à titre d'exemple, d'un mode préféré de réalisation de l'invention. On se reportera à la figure unique annexée qui est un schéma d'une installation selon l'invention de lavage d'air pollué ou d'effluents gazeux provenant d'une cuve de traitement ou d'une cuve de séjour de produits ou de déchets organiques nauséabonds. Ce schéma n'a pas d'échelle définie.

De l'air pollué à traiter est évacué à l'atmosphère par un exhausteur 1 à travers une canalisation 2, un laveur à ruissellement 3 et une autre canalisation 4. Au sommet du laveur à ruissellement 3, une solution aqueuse provenant d'un bac 25 étanche aux gaz est amenée par une pompe 6 à travers une canalisation 7 et des pulvérisateurs 24. De la base du laveur part une canalisation 5 qui recycle la solution aqueuse au bac 25. Une source d'ozone 10 amène de l'ozone au choix à l'une des deux canalisations 12 ou 13 au moyen respectivement d'une vanne 15 et d'une vanne 16. La canalisation 13 permet d'amener l'ozone à un piquage 22, sur la canalisation 2, c'est-à-dire dans l'air pollué à traiter. La canalisation 12 permet d'amener l'ozone à un piquage 23 au fond du bac 25 pour l'y faire barboter dans la solution aqueuse. Du sommet du bac 25 part une canalisation 8 qui conduit à un piquage 9 sur la canalisation 2, c'est-à-dire également dans l'air pollué à traiter. On voit que, dans le premier cas, l'ozone est introduit dans la phase gazeuse, où il va agir activement, mais que l'ozone non consommé rencontrera à contre-courant la solution aqueuse dans le laveur 3 pour y être absorbé dans la phase liquide. Dans le second cas, l'ozone enrichit d'abord la phase liquide par absorption par barbotage, puis agit ensuite directement sur la phase gazeuse à épurer.

Selon un mode de réglage simple, on peut lier la quantité d'ozone introduit à la quantité de produits soufrés réduits contenus dans les gaz. Une quantité équimoléculaire d'ozone est suffisante. Mais, selon un mode de réglage préféré, on prélève sur la conduite 4, par un piquage 17, un échantillon d'air épuré, qu'on envoie par une conduite 18 à un analyseur 19, par exemple à absorption UV, dosant l'ozone résiduel. Un signal de sortie de l'analyseur est envoyé à un dispositif de régulation 20 qui, par une ligne de liaison 21, envoie un ordre de réglage à une vanne réglable 14 interposée sur la conduite 11. De cette façon on peut régler le débit d'ozone introduit par asservissement à une valeur de consigne, par exemple 0,1 ppm, de la quantité d'ozone dosée dans l'air effluent sortant du laveur 3 par la conduite 4.

On remarquera qu'aucun moyen de chauffage n'est prévu, l'ozone étant introduit à la température ambiante et la solution aqueuse elle-même pouvant être à la température ambiante.

Avec l'installation représentée, le titulaire a procédé à des essais, à la suite desquels il a établi le rendement d'épuration pour différents polluants identifiés et pour quatre types de lavage, à savoir:

A) lavage à l'eau du réseau (eau perdue),
B) lavage à l'eau oxygénée à 5% en masse,
C) lavage à l'eau du réseau (eau perdue) + ozone, comme il est connu en soi,
D) lavage selon le procédé de l'invention.

Les mesures de rendement exprimées en pourcent de taux d'épuration sont récapitulées sur le tableau ci-après.

| Polluant | Type de lavage | | | |
|---|---|---|---|---|
| | A (%) | B (%) | C (%) | D (%) |
| Hydrogène sulfuré | 0,5 | 54 | ~100 | ~100 |
| Méthylmercaptan | 2 | 7,2 | 60 | 68 |
| Acroléine | 47,5 | 82 | 47,5 | 82 |
| Triméthylamine | 97 | 97 | 97 | 97 |
| Ammoniac | ~100 | ~100 | ~100 | ~100 |
| Méthyléthylcétone | 75,2 | 79 | 75,2 | 79 |
| Valéraldéhyde | 6,6 | 84 | 6,6 | 84 |
| Butyraldéhyde | 43 | 94 | 43 | 94 |

Ce qui est le plus surprenant n'est pas tant le taux de rendement que son obtention sans addition de peroxyde d'hydrogène autre que les 5% en masse initiaux. Ce qui est également très surprenant, c'est la quantité très faible, pratiquement stœchiométrique, de consommation d'ozone permise par le procédé de l'invention.

**Revendications**

1. Procédé de désodorisation d'air pollué par des gaz malodorants d'origine organique comprenant l'utilisation d'ozone et le lavage de cet air à l'aide d'une solution aqueuse de peroxyde d'hydrogène, caractérisé en ce qu'on introduit l'ozone dans un circuit fermé de la solution de lavage sans appoints de peroxyde d'hydrogène, pour obtenir et maintenir à la concentration voulue en peroxyde d'hydrogène ladite solution de lavage, cette introduction d'ozone se faisant soit directement dans ladite solution, soit par l'intermédiaire de l'air à traiter venant en contact avec la solution de lavage.

2. Procédé de désodorisation d'air pollué contenant des gaz malodorants d'origine organique selon la revendication 1, caractérisé en ce qu'on introduit l'ozone directement dans la solution aqueuse de lavage, avant exécution de l'opération de lavage de l'air pollué.

3. Procédé selon l'une des revendications 1 ou 2, caractérisé en ce que l'ozone est introduit à la température ambiante et la solution aqueuse de lavage est elle-même à la température ambiante.

4. Procédé selon la revendication 2, caractérisé en ce que le rapport massique initial du peroxyde d'hydrogène dans la solution est compris entre 0,5/100 et 10/100 et de préférence de l'ordre de 5/100.

5. Procédé selon la revendication 2, caractérisé en ce qu'on introduit l'ozone par barbotage dans la solution aqueuse de lavage séjournant dans un réservoir-tampon ou bac étanche aux gaz (25).

6. Procédé selon la revendication 2, caractérisé en ce que, après introduction de l'ozone dans la solution aqueuse de lavage, on récupère la phase gazeuse non absorbée et on l'introduit dans l'air pollué avant son lavage par ladite solution aqueuse.

7. Procédé selon l'une des revendications 1 ou 6, caractérisé en ce qu'on introduit l'ozone en quantité équimoléculaire de la quantité de produits soufrés réduits contenus dans l'air à désodoriser.

8. Procédé selon la revendication 7, caractérisé en ce qu'on règle le débit d'ozone introduit par asservissement à une valeur de consigne de la quantité d'ozone dosée dans l'air effluent sortant du laveur (3).

## Patentansprüche

1. Verfahren zum Desodorisieren von Luft, die durch übelriechende Gase organischen Ursprungs verschmutzt ist, unter Verwendung von Ozon und Waschung dieser Luft mit Hilfe einer wässerigen Lösung von Wasserstoffperoxid, dadurch gekennzeichnet, dass man das Ozon in einen geschlossenen Kreislauf der Waschlösung ohne zusätzliches Wasserstoffperoxid einleitet, um die Waschlösung zu erhalten und auf der gewollten Wasserstoffperoxidkonzentration zu halten, wobei die Einleitung des Ozons entweder direkt in die Lösung oder durch die zu behandelnde Luft erfolgt, die in Kontakt gelangt mit der Waschlösung.

2. Verfahren zum Desodorisieren von verschmutzter Luft, die ein übelriechendes Gas organischen Ursprungs enthält, nach Anspruch 1, dadurch gekennzeichnet, dass man das Ozon direkt in die wässerige Waschlösung vor der Ausführung des Waschvorgangs der verschmutzten Luft einleitet.

3. Verfahren nach einem der Ansprüche 1 oder 2, dadurch gekennzeichnet, dass das Ozon bei Umgebungstemperatur eingeleitet wird, und dass die wässerige Waschlösung ebenfalls Umgebungstemperatur aufweist.

4. Verfahren nach Anspruch 2, dadurch gekennzeichnet, dass das anfängliche Massenverhältnis des Wasserstoffperoxids in der Lösung im Bereich von 0,5/100 bis 10/100 und vorzugsweise in der Grössenordnung von 5/100 liegt.

5. Verfahren nach Anspruch 2, dadurch gekennzeichnt, dass man das Ozon mittels Durchblasen in die wässerige Waschlösung, die sich in einem Pufferbehälter (25) oder in einem gasdichten Behälter befindet, einleitet.

6. Verfahren nach Anspruch 2, dadurch gekennzeichnet, dass man nach Einleitung des Ozons in die wässerige Waschlösung die gasförmige, nicht absorbierte Phase rückgewinnt, und dass diese Phase in die verschmutzte Luft vor deren Waschung mit der wässerigen Lösung eingeleitet wird.

7. Verfahren nach einem der Ansprüche 1 bis 6, dadurch gekennzeichnet, dass man das Ozon in einer Menge einleitet, die äquimolekular ist der Menge der reduzierten schwefelhaltigen Produkte, die in der zu desodorierenden Luft enthalten sind.

8. Verfahren nach Anspruch 7, dadurch gekennzeichnet, dass man den Durchsatz des eingeleiteten Ozons durch Regelung auf einen Einstellwert der Ozonmenge regelt, die in der ausströmenden Luft, die aus dem Reinigungsgefäss (3) austritt, quantitativ bestimmt wird.

## Claims

1. A process for deodorising air which is polluted by malodorous gases of organic origin comprising the use of ozone and washing of this air with an aqueous solution of hydrogen peroxide, characterised in that the ozone is introduced into a closed circuit of the washing solution which is not topped up with hydrogen peroxide, so as to obtain and maintain the said washing solution at the desired concentration of hydrogen peroxide, the said introduction of ozone being made either directly into the said solution or by the intermediary of the air which is to be treated coming into contact with the washing solution.

2. A process for deodorisation of polluted air containing malodorous gases of organic origin according to Claim 1, characterised in that the ozone is introduced directly into the aqueous washing solution, before carrying out the operation of washing the polluted air.

3. A process according to Claim 1 or 2, characterised in that the ozone is introduced at ambient temperature and the aqueous washing solution is itself at ambient temperature.

4. A process according to Claim 2, characterised in that the initial weight ratio of hydrogen peroxide in the solution is between 0.5/100 and 10/100 and preferably of the order of 5/100.

5. Process according to Claim 2, characterised in that the ozone is introduced by bubbling it into the aqueous washing solution which is held in a buffer reservoir or gas-tight tank (25).

6. A process according to Claim 2, characterised in that after introduction of the ozone into the aqueous washing solution, the non-absorbed gas phase is recovered and is introduced into the polluted air before the air is washed with the said aqueous solution.

7. A process according to Claim 1 or 6, characterised in that the ozone is introduced in an amount equimolecular with the amount of reduced sulphur products contained in the air to be deodorised.

8. A process according to Claim 7, characterised in that the flow rate of ozone is regulated by feedback control as a function of a target value of the amount of ozone determined in the effluent air leaving the washer (3).